# FASCICULE DE BREVET EUROPEEN

(11) **EP 1 932 510 B1**
(45) Date de publication et mention de la délivrance du brevet: **16.03.2016**
(21) Numéro de dépôt: 06291929.5
(22) Date de dépôt: 14.12.2006
(51) Int. Cl.: A61K 8/36, A61K 8/97, A61Q 5/00, A61Q 19/00

(54) **Utilisation d'acide pétrosélinique pour le traitement des cuirs chevelus fragiles**
Verwendung der Petroselinsäure zur Behandlung der fragilen Kopfhaut
Use of petroselinic acid for the treatment of fragile scalps

(43) Date de publication de la demande: 18.06.2008
(73) Titulaire: L'Oréal, 75008 Paris (FR); Nestec S.A., 1800 Vevey (CH)
(72) Inventeur: Gueniche, Audrey, 92500 Rueil Malmaison (FR); Castiel, Isabelle, 06200 Nice (FR)
(74) Mandataire: Le Coupanec, Pascale A.M.P.

(56) Documents cités:
- EP-A- 0 116 439
- EP-A- 0 888 773
- EP-A1- 1 013 178
- EP-A2- 0 709 084
- WO-A-01/08651
- WO-A-99/47110
- WO-A-02/102337
- DE-A1- 10 035 735
- US-A- 6 022 896
- TAKEUCHI Y ET AL: "SKIN PENETRATION ENHANCING ACTION OF CIS-UNSATURATED FATTY ACIDS WITH OMEGA-9, AND OMEGA-12-CHAIN LENGTHS" BIOLOGICAL & PHARMACEUTICAL BULLETIN (OF JAPAN), PHARMACEUTICAL SOCIETY OF JAPAN, TOKYO, JP, vol. 21, no. 5, mai 1998 (1998-05), pages 484-491, XP000755077 ISSN: 0918-6158
- COMITÉ DE PROMOTION ET DE GESTION DE LA SANTÉ DE L'INDUSTRIE DE LA CONSTRUCTION (CANADA): POUR UNE INDUSTRIE EN SANTE, [Online] vol. 3, no. 2, juin 2004 (2004-06), XP002438123 Extrait de l'Internet: URL:http://www.bibliotheque.assnat.qc.ca/0 1/PER/779693/2004/Vol_3_no_2_(juin_2004).p df> [extrait le 2007-06-18]

## Description

La présente invention concerne l'utilisation d'acide pétrosélénique pour le soin des cuirs chevelus fragiles, réactifs et/ou sensibilisés.

La peau humaine est constituée de deux compartiments, à savoir un compartiment profond, le derme et un compartiment superficiel, l'épiderme.

L'épiderme est en contact avec l'environnement extérieur. Un de ses rôles consiste à protéger l'organisme de la déshydratation et des agressions extérieures, notamment liées à des facteurs environnementaux de type agents irritants ou polluants (détergents, pollution, fumée de cigarettes...), sollicitations mécaniques (frottements, abrasion, rasage, lavage fréquent...), déséquilibres thermiques ou climatiques (froid, vent, sécheresse, radiations UV...), xénobiotiques (micro-organismes, allergènes...), traitements chimiques cosmétiques ou dermatologiques (peeling, traitement anti-acné...), ou par des facteurs physiologiques (âge, stress...).

Il est composé principalement de trois types de cellules qui sont les kératinocytes, très majoritaires, les mélanocytes et les cellules de Langerhans, qui sont délimitées par une structure lipidique intercellulaire. Cette structure lipidique ainsi que la cohésion des cellules épidermiques participent aux échanges en eau de la peau et à la fonction protectrice ou 'fonction barrière' de la peau, d'une part vis-à-vis des déperditions en eau (perte insensible en eau) et d'autre part vis-à-vis des agressions extérieures.

La cohésion des cellules épidermiques de la peau et/ou des semi-muqueuses peut être altérée notamment par des facteurs physiologiques internes comme l'âge, le stress, des changements hormonaux et/ou par des facteurs environnementaux tels que des déséquilibres thermiques ou climatiques, des xénobiotiques, des agents irritants, des sollicitations mécaniques de type frottements, lavage, rasage....

La peau et/ou les semi-muqueuses se fragilisent alors et deviennent plus sujettes aux crevasses et aux gerçures, moins résistantes aux frictions mécaniques et plus perméables à l'environnement extérieur et aux bactéries.

Sur la peau et/ou les lèvres, l'apparition de microgerçures peut avoir pour conséquence une moins bonne homogénéité du maquillage appliqué sur celles-ci (ex : fond de teint, rouge à lèvres), ce qui peut ne pas être esthétique.

On assiste également à une désorganisation générale de la structure cutanée liée à une diminution de la cohésion entre les cellules, tant au niveau de l'épiderme que du derme.

Pour protéger la peau et/ou des semi-muqueuses, une solution traditionnelle consiste à apporter en permanence des agents externes lipidiques (ex : corps gras, céramides) et/ou des agents favorisant la synthèse de lipides épidermiques (ex : vitamine C et ses dérivés) et/ou des agents émollients ou hydratants.

Mais il subsiste toujours le besoin de trouver de nouvelles compositions permettant d'améliorer la cohésion cellulaire et/ou renforcer la cohésion de la structure du cuir chevelu et traiter ainsi le cuir chevelu fragilisé, sensibilisé, stressé et/ou réactif.

De manière inattendue, la Demanderesse a constaté que l'acide pétrosélinique pouvaient s'avérer efficace pour le traitement des cuirs chevelus sensibilisés, réactifs, stressés et/ou fragilisées.

Les acides gras mono-insaturés ont été décrits pour différentes applications telles que l'hydratation de la peau sèche (EP 0 709 084 de L'Oreal).

EP 0 116 439 (Suntory) divulgue que certains acides gras ont une forte activité d'inhibition de la 5α-réductase de la flore bactérienne du cuir chevelu, et peuvent être utilisés de ce fait dans les produits toniques pour le traitement des cheveux. L'action de ces acides gras est donc limitée à la flore bactérienne du cuir chevelu.

US 4,097,604 (Oxford Hill) indique qu'un sel de différents acides gras est actif contre les pathogènes de la cavité buccale, permettant ainsi de diminuer l'incidence de ces bactéries sur d'apparition de periodontoses. L'action de ces acides gras est donc limitée à la flore buccale.

EP 0 355 842 (Sansho Seiyaku) décrit une crème de soin destinée à prévenir la pigmentation causée par une surproduction de mélanine, ladite crème pouvant renfermer de l'acide pétrosélinique.

EP 0 116 439 (Suntory) décrit des lotions capillaires comprenant au moins un acide gras pour le traitement des pellicules et des démangeaisons du cuir chevelu.

EP 1 013 178 (Unilever) décrit l'utilisation d'acide pétrosélinique comme agent anti-inflammatoire et pour traiter les signes cutanés du vieillissement (ride, peau flasque, taches de sénescence).

Cependant, aucun de ces documents ne laissait présager l'activité des acides gras monoinsaturés sur la cohésion de tissu cutané et la qualité de sa surface.

Ainsi la présente invention concerne selon un premier de ses aspects l'utilisation d'au moins l'acide pétrosélinique, de ses sels et/ou esters, pour renforcer la cohésion du tissu cutané du cuir chevelu.

La Demanderesse a en effet pu mettre en évidence que les acides gras monoinsaturés conduisaient à préserver le tissu cutané sous-jacent et à améliorer ainsi la qualité de la peau et à corriger sa fragilité :
- une restructucturation du réseau collagénique notamment visible par coloration au Trichorme Masson sur une coupe de peau d'un individu supplémenté avec de l'huile de coriandre, en effet, cette huile a comme activité la diminution de l'activité collagénase et élastase (mesuré par dosage enzymatique) ;
- une diminution des marqueurs de stress et d'inflammation (tels que Hsp 70 et TNF-alpha dont la mesure peut être réalisée avec des anti-corps spécifiques) qui participent à la réactivité et à la sensibilité cutanée et exacerbent la fragilité de la peau et des muqueuses.

### Acide pétrosélinique

L'acide gras mono-insaturé utilisé dans le cadre de la présente invention est l'acide pétrosélinique (ou acide delta-6-cis-octadecenoique en C18).

Lorsqu'il se présente sous forme de sel, l'acide gras mono-insaturé peut plus particulièrement être un sel cosmétiquement acceptable, c'est-à-dire, un sel inorganique tel qu'un sel d'ammonium ou de métaux alcalins (lithium, potassium, sodium), d'alcalino-terrreux (magnésium, calcium) ou un sel d'aluminium. En particulier, l'acide gras mono-insaturé se présente sous forme de sel de calcium.

Lorsqu'il se présente sous forme d'ester, ainsi, l'acide gras mono-insaturé peut être estérifié avec le glycérol (mono-, di- ou tri-acyl), un alcool comme les alcools méthylique et éthylique, un sucre, un tocophérol, un tocotriénol, un stérol ou encore un alcool gras.

Selon une variante de l'invention, l'acide gras moni-insaturé est utilisé sous une forme isolée, c'est-à-dire après extraction de sa source d'origine.

Selon une autre variante de l'invention, l'acide gras mono-insaturé provient d'extraits végétaux tels que des huiles.

Ainsi l'invention se rapporte notamment à l'utilisation d'une huile riche en acide gras mono-insaturé.

Les huiles riches en acide pétrosélinique sont plus particulièrement choisies parmi les huiles d'ombellifère.

On entend par huile riche en acide pétrosélinique, une huile comprenant au moins 40% d'acide prétrosélinique.

Les ombellifères sont des plantes dont les fleurs sont disposées en ombelles, les espèces particulièrement riches en acide pétrosélénique sont les Umbellifarea-Apiacea et Araliaceae. Les plantes du genre Thapsia sont également des sources d'acide pétrosélénique (Avato et al, Lipids, 2001, 36, 845). Les espèces de préférence utilisées dans l'invention sont le coriandre, le cerfeuil, la carotte, le céleri, le cumin, le carvi, le persil et l'aneth. L'huile d'ombellifère utilisée selon l'invention peut être extraite de la graine de cette ombellifère, par exemple par broyage ou pressage, puis raffinage. L'huile d'ombellifère a une teneur en acide pétrosélinique qui varie selon la graine d'ombellifère d'où elle est extraite. Pour une même ombellifère, la teneur en acide pétrosélinique varie aussi selon le pays d'origine de l'ombellifère et selon l'extraction qui peut être plus ou moins complète.

L'acide pétrosélénique est également un composé abondant (environ 48%) de l'huile de graine de Géranium sanguineum **(**Tsevegsuren et al, Lipids, 2004, 39, 571**).**

L'administration des composés selon l'invention se fait par voie orale.

La teneur en acide gras ou ester d'acide gras monoinsaturé dans les compositions pour la voie orale sera telle que les doses journalières sont comprises entre 0,5 et 2500 mg/j, notamment entre 5 et 500 mg/j.

L'utilisation selon l'invention permet d'améliorer l'état du cuir chevelu.

### Applications

L'utilisation selon l'invention permet aussi de lisser la surface du cuir chevelu et/ou prévenir et/ou traiter les rugosités et/ou les irrégularités du relief de la peau.

En particulier, l'utilisation selon l'invention est destinée à renforcer la résistance mécanique du cuir chevelu, en particulier d'agressions extérieures choisies parmi les facteurs environnementaux de type agents irritants ou polluants ; sollicitations mécaniques ; déséquilibres thermiques ou climatiques ; xénobiotiques ; traitements chimiques cosmétiques ou dermatologiques ou par des facteurs physiologiques.

L'utilisation selon l'invention permet encore de prévenir et/ou traiter les peaux fragilisées, sensibilisée et/ou réactives.

En particulier, l'acide pétrosélinique est utilisé pour:
- prévenir et/ou diminuer la fragilisation de la peau et/ou protéger la peau contre les agressions extérieures, notamment liées à des facteurs environnementaux (de type agents irritants ou polluants (détergents, pollution, fumée de cigarettes...), sollicitations mécaniques (frottements, abrasion, rasage) déséquilibres thermiques ou climatiques (froid, sécheresse, radiations), xénobiotiques (micro-organismes, allergènes), traitements chimiques cosmétiques ou dermatologiques (peeling, traitement anti-acné...), ou par des facteurs physiologiques (âge, stress...) ; et/ou rendre la peau plus résistante aux agressions extérieures ; et/ou
- améliorer le confort de la peau, en particulier prévenir et/ou diminuer les tiraillements, les picotements ; et/ou
- améliorer l'aspect de surface de la peau, en particulier la rugosité et/ou les irrégularités de relief.

Les altérations consécutives à un défaut de cohésion des cellules de la peau peuvent aussi survenir chez des sujets dits à peau sensible et ainsi amplifier les altérations se manifestant classiquement chez les sujets à peau sensible.

D'une manière générale, les peaux sensibles se définissent par une réactivité particulière de la peau. Toutefois, par opposition aux peaux qualifiées d'allergiques, cette réactivité ne relève pas d'un processus immunologique, c'est-à-dire ne se produit pas uniquement sur une peau déjà sensibilisée en réponse à la présence d'un allergène. Son mécanisme est dit aspécifique.

Cette réactivité cutanée se traduit classiquement par la manifestation de signes d'inconfort en réponse à la mise en contact du sujet avec un élément déclenchant qui peut avoir diverses origines. Il peut s'agir de l'application d'un produit cosmétique en surface de la peau sensible, de la prise d'aliments, de l'exposition à des variations brutales de températures, à la pollution atmosphérique et/ou à des rayons aux ultra-violets ou infrarouges. Il existe également des facteurs associés comme l'âge et le type de peau. Ainsi les peaux sensibles sont plus fréquentes parmi les peaux sèches ou grasses que parmi les peaux normales.

L'apparition de ces signes d'inconfort, qui apparaissent dans les minutes qui suivent la mise en contact du sujet avec l'élément déclenchant, est une des caractéristiques essentielles des peaux sensibles. Il s'agit principalement de sensations dysesthésiques. On entend par sensations dysesthésiques, des sensations plus ou moins douloureuses ressenties dans une zone cutanée comme les picotements, fourmillements, démangeaisons ou prurits, brûlures, échauffements, inconforts, tiraillements, etc. Ces signes subjectifs existent le plus souvent en l'absence de signes chimiques visibles tels que la rougeur et les desquamations. On sait aujourd'hui que ces réactions d'irritation et d'intolérance cutanée sont notamment liées à une libération de neuropeptides par les terminaisons nerveuses de l'épiderme et du derme.

Un test permettant d'identifier les sujets à peau sensible est décrit dans l'EP 1 374 913. Au sens de la présente invention, les peaux sensibles couvrent les peaux irritables et les peaux intolérantes.

Une peau intolérante est une peau qui réagit par des sensations d'échauffement, de tiraillements, de fourmillements et/ou de rougeurs, à différents facteurs tels que l'application de produits cosmétiques ou dermatologiques ou de savon. En général, ces signes sont associés à un érythème et à une peau hyper-séborrhéique ou acnéique, voire même rosacéiforme, avec ou sans dartres.

Une peau irritable est une peau qui réagit par un prurit, c'est-à-dire par des démangeaisons ou par des picotements, à différents facteurs tels que l'environnement, les émotions, les aliments, le vent, les frottements, le rasoir, l'eau dure à forte concentration de calcaire, les variations de température ou la laine.

En renforçant la cohésion des cellules de la peau, l'utilisation selon l'invention contribue à traiter les peaux sensibles.

### Galénique

Les compositions selon l'invention sont administrées par voie orale.

Les compositions selon l'invention peuvent se présenter sous toutes les formes galéniques normalement utilisées selon la voie d'utilisation.

Pour l'ingestion, de nombreuses formes de réalisation de compositions orales et notamment de compléments alimentaires sont possibles. Leur formulation est réalisée par les procédés usuels pour produire des dragées, gélules, gels, émulsions, comprimés, capsules ou solutions.

En particulier, le(s) actif(s) selon l'invention peuvent être incorporés dans toutes autres formes de compléments alimentaires ou d'aliments enrichis, par exemple des barres alimentaires, ou des poudres compactées ou non. Les poudres peuvent être diluées à l'eau, dans du soda, des produits laitiers ou dérivés du soja, ou être incorporées dans des barres alimentaires.

Les actifs selon l'invention peuvent être formulés avec les excipients et composants usuels pour de telles compositions orales ou compléments alimentaires, à savoir notamment composants gras et/ou aqueux, agents humectants, épaississants, conservateurs, agents de texture, de saveur et/ou d'enrobage, antioxydants, conservateurs et colorants usuels dans le domaine de l'alimentaire.

Les agents de formulation et excipients pour composition orale, et notamment pour compléments alimentaires sont connus dans ce domaine et ne font pas ici l'objet d'une description détaillée.

Conviennent notamment comme supports alimentaires ou pharmaceutiques, le lait, le yaourt, le fromage, les laits fermentés, les produits fermentés à base de lait, des glaces, des produits à base de céréales fermentées, des poudres à base de lait, des formules pour enfants et nourrissons, des aliments pour animaux en particulier domestiques, des comprimés ou tablettes, des suspensions de bactéries liquides, des suppléments oraux sous forme sèche et les suppléments oraux sous forme liquide.

En particulier, la composition selon l'invention peut être une composition alimentaire pour la consommation humaine. Il peut s'agir en particulier d'aliments complets nutritionnels, de boissons, d'eaux minérales, de soupes, de suppléments diététiques et d'aliments de remplacement, de barres nutritionnelles, de confiserie, de produits à base de lait ou à base de lait fermenté, de yaourts, de poudres à base de lait, de produits de nutrition entérale, de compositions pour enfants et/ou nourrissons, de produits à base de céréales ou de produits à base de céréales fermentées, de glaces, de chocolat, de café, de produits « culinaires » tels que de la mayonnaise, de la purée de tomate ou des assaisonnements pour salades.. La composition selon l'invention peut également être destinée aux animaux.

Ces compositions sont préparées selon les méthodes usuelles.

### Associations

Les acides gras mono-insaturés utilisables selon l'invention sont avantageusement associés à des actifs.

A titre d'actifs utilisables, on peut citer, les vitamines B3, B5, B6, B8, C, E, ou PP, les caroténoïdes, les curcuminoïdes et la niacine.

En particulier, on peut utiliser un complexe anti-oxydant comprenant les vitamines C et E, et au moins un caroténoïde, notamment un caroténoïde choisi parmi le β-carotène, le lycopène, l'astaxanthine, la zéaxanthine et la lutéine, des flavonoïdes telles que les catéchines, l'hespéridine, des proanthocyanidines et des anthocyanines.

La composition peut contenir un ou plusieurs cation(s) minéral(aux) divalent(s) sous différentes formes. Le cation minéral divalent peut ainsi être sous la forme d'un sel minéral ou organique, anhydre ou hydraté ou d'un complexe chelaté. Ces sels peuvent être par exemple des carbonates, des bicarbonates, des sulfates, des glycérophosphates, des chlorures, des nitrates, des acétates, des hydroxydes, des oxydes, des sels d'α-hydroxyacides (citrates, tartrates, lactates, malates) ou d'acides de fruits, ou encore des sels d'acides aminés (aspartate, arginate, fumarate) ou des sels d'acides gras (palmitate, oléate, caséinate, béhénate). Le cation minéral divalent est choisi parmi le manganèse, le cuivre et/ou le zinc ou parmi les alcalino-terreux. Comme métal alcalino-terreux utilisable dans l'invention, on peut citer le baryum, le calcium, le magnésium, le strontium et/ou le béryllium. Avantageusement, le cation minéral divalent et notamment métal alcalino-terreux est utilisé dans la présente invention sous forme de sel. En particulier, le sel peut être choisi parmi les sels de nitrate, citrate, chlorure, gluconate, sulfate, lactate et/ou acétate. Le cation minéral divalent peut aussi être utilisé sous la forme d'un complexe chélaté notamment à des protéines cristallisées ou ionisées. Le cation minéral divalent peut encore être sous une forme spécifique stockée par un microorganisme, par exemple de type levure, à l'image des levures séléniées.

Il est possible d'utiliser avantageusement dans les mélanges des extraits bactériens parmi les bactéries filamenteuses non photosynthétiques et non fructifiantes telles que définies selon la classification du Bergey's Manual of Systemic Bacteriology, volume 3, section 23, 9ème édition 1989. On citera plus particulièrement les bactéries appartenant à l'ordre des Beggiatoales, et notamment les bactéries appartenant au genre *Beggiotoa.* On peut citer par ailleurs les bactéries appartenant au genre *Vitreoscilla,* proche du genre *Beggiatoa.* Parmi les bactéries utilisables, on peut citer par exemple, *Vitreoscilla beggiatoïdes* (ATCC 43181) et *Beggiatoa alba* (ATCC33555), et préférentiellement l'utilisation de l'extrait de *Vitreoscilla filiformis* avec en particulier la souche ATCC 15551, ses métabolites et ses fractions pourront être utilisés.

La composition comprend aussi avantageusement au moins un probiotique, un prébiotique ou un mélange de probiotiques et un mélange de prébiotiques.

Des exemples spécifiques de microorganismes probiotiques sont *Bifidobacterium adolescentis, Bifidobacterium animalis, Bifidobacterium bifidum, Bifidobacterium breve, Bifidobacterium lactis, Bifidobacterium longum, Bifidobacterium infantis, Bifidobacterium pseudocatenulatum, Lactobacillus acidophilus (LC1, NCFB 1748) ; Lactobacillus amylovorus, Lactobacillus casei (Shirota), Lactobacillus rhamnosus* (souche GG), *Lactobacillus brevis, Lactobacillus crispatus, Lactobacillus delbrueckii (subsp bulgaricus, lactis), Lactobacillus fermentum, Lactobacillus helveticus, Lactobacillus gallinarum, Lactobacillus gasseri, Lactobacillus johnsonii, Lactobacillus paracasei, Lactobacillus plantarum, Lactobacillus reuteri, Lactobacillus rhamnosus, Lactobacillus salivarius), Lactobacillus alimentarius, Lactobacillus curvatus, Lactobacillus casei subsp. casei, Lactobacillus sake Lactococcus lactis, Enterococcus (faecalis, faecium), Lactococcus lactis (subspp lactis ou cremoris), Leuconstoc mesenteroides* subsp *dextranicum, Pediococcus acidilactici, Sporolactobacillus inulinus, Streptococcus salvarius subsp. Thermophilus, Streptococcus thermophilus, Staphylococccus carnosus, Staphylococcus xylosus, Saccharomyces (cerevisiae* ou encore *boulardii), Bacillus (cereus var toyo ou subtilis), Bacillus coagulans, Bacillus licheniformis, Escherichia coli strain nissle, Propionibacterium freudenreichii et leurs mélanges.*

Les microorganismes peuvent être formulés à l'état de poudres, c'est-à-dire sous une forme sèche, ou sous forme de suspensions ou de solutions.

Plus particulièrement, il s'agit de microorganismes probiotiques issus du groupe des bactéries lactiques, comme notamment les *Lactobacillus* et/ou les *Bifidobacterium.* A titre illustratif de ces bactéries lactiques, on peut plus particulièrement citer les *Lactobacillus johnsonii, Lactobacillus reuteri, Lactobacillus rhamnosus, Lactobacillus paracasei, Lactobacillus casei* ou *Bifidobacterium bifidum, Bifidobacterium breve, Bifidobacterium longum, Bifidobacterium animalis, Bifidobacterium lactis, Bifidobacterium infantis, Bifidobacterium adolescentis* ou *Bifidobacterium pseudocatenulatum* et leurs mélanges. Les espèces convenant tout particulièrement sont les *Lactobacillus johnsonii, Lactobacillus paracasei, Bifidobacterium adolescentis, Bifidobacterium longum* et *Bifidobacterum Lactis NCC 2818 (encore désigné Bb12 ATCC* 27536) respectivement déposées suivant le traité de Budapest avec l'Institut Pasteur (28 rue du Docteur Roux, F-75024 Paris cedex 15) les 30/06/92, 12/01/99, 15/04/99, 15/04/99, 07/06/05 sous les désignations suivantes CNCM I-1225, CNCM I-2116, CNCM I-2168 et CNCM I-2170 et CNCM I-3446, et le genre *Bifidobacterium longum (BB536).* La souche de *Bifidobacferium lactis* CNCM I-3446 peut être obtenue chez Hansen (Chr. Hansen A/S, 10-12 Boege Alle, P.O. Box 407, DK-2970 Hoersholm, Danemark).

Selon un mode de réalisation particulier de l'invention, la composition comprend au moins deux microorganismes, notamment probiotiques et/ou métabolites et/ou fractions différents. Ces microorganismes peuvent différer par leur nature par exemple bactérie et champignon, ou bien encore par leur famille, leur genre, leur espèce, ou seulement par leur souche.

Plus particulièrement, les prébiotiques peuvent être choisis parmi les oligosaccharides, produits à partir du glucose, galactose, xylose, maltose, sucrose, lactose, amidon, xylane, l'hémicellulose, l'inuline, des gommes, de type acacia par exemple, ou un de leurs mélanges. Plus particulièrement, l'oligosaccharide comprend au moins un fructo-oligosaccharide. Plus particulièrement, ce prébiotique peut comprendre un mélange de fructo-oligosaccharide et d'inuline.

La composition peut contenir aussi avantageusement Les acides gras polyinsaturés comprennent notamment les acides gras ω-3 et les acides gras w-6, caractérisés par la position de l'insaturation la plus proche du groupe méthyle terminal et plus particulièrement à l'invention les acides gras insaturés comportant de 18 à 22 atomes de carbone, en particulier les acides gras polyinsaturés, notamment les acides gras ω-3 et ω-6.

Parmi les acides gras polyinsaturés de la série ω-6 utilisable dans la composition, on peut citer en particulier l'acide linoléique, à 18 atomes de carbone et deux insaturations (18:2, ω-6), l'acide γ-linolénique à 18 atomes de carbone et trois insaturations (18:3, w-6), l'acide di-homogamalinolénique à 20 atomes de carbone et 3 insaturations (20:3, w-6), l'acide arachidonique, l'acide 5, 8, 11, 14 éicosatétraénoïque (20:4, w-6) et l'acide docosatétraenoique (22:4, w-6).

Les acides gras polyinsaturés de la série w-3 peuvent notamment être choisis parmi l'acide α-linolénique (18:3, w-3), l'acide stéaridonique (18:4, w-3), l'acide 5,8,11,14,17-eicosapentaénoïque ou EPA (20:5, ω-3), et l'acide 4,7,10,13,16,19-docosahéxaénoïque ou DHA (22:6, w-3), l'acide docosapentanoique (22,5, w-3), l'acide n-butyl-5,11,14-eicosatrienonique. Et tout particulièrement à l'inventionl'acide α-linolénique, l'acide γ-linolénique, l'acide stéaridonique, l'acide éicosapentaénoïque, l'acide docosahexaénoïque, leurs mélanges ou les extraits les comportant.

Les sources d'acide γ-linolénique peuvent être choisies parmi les huiles végétales comme par exemple les huiles d'onagre, de bourrache, de pépin de cassis, d'echium et de chanvre, et les extraits de la microalgue spiruline (*Spirulina maxima* et *Spirulina platensis).*

Les huiles végétales de noix, noisettes, amandes *(Juglans regia), de coriandre* et de soja (*Glycina max*)*,* de colza (*Brassica naptus*), de chia, de lin, de rosier muscat et les huiles de poisson, par exemple, sont riches en acides gras polyinsaturés de la série ω-3. Les acides gras polyinsaturés w-3 peuvent également se trouver dans le zooplancton, les crustacés/mollusques et les poissons. Les huiles de poissons constituent la principale source industrielle d'EPA et de DHA. Les biomasses de micro algues peuvent aussi constituer une matière première d'extraction des acides gras insaturés ω-3. Ainsi, l'acide gras insaturé peut être mis en oeuvre dans la composition sous forme d'au moins une huile choisie parmi les huiles d'onagre, de bourrache, de pépins de cassis, de noix, de soja, de poissons, de tournesol, de germes de blé, de chanvre, de fénugrec, de rosier muscat, d'échium, d'argan, de baobab, de son de riz, de sésame, d'amande, de noix, de noisette, de chia, de lin, de rosier muscat, d'olive, d'avocat, de carthame, de coriandre et/ou d'extrait de biomasse microalgues (par exemple spiruline), ou d'extraits de zooplancton.

Comme actifs hydrophiles, on peut utiliser les protéines ou les hydrolysats de protéine, les acides aminés, les polyols notamment en C₂ à C₁₀ comme les glycérine, sorbitol, butylène glycol et polyéthylène glycol, l'urée, l'allantoïne, les sucres et les dérivés de sucre, les vitamines hydrosolubles, l'amidon, des extraits bactériens ou végétaux comme ceux d'Aloe Vera.

Comme actifs lipophiles, on peut utiliser le rétinol (vitamine A) et ses dérivés, le tocophérol (vitamine E) et ses dérivés, les céramides, les huiles essentielles.

On peut, en outre, associer les actifs selon l'invention, à des agents actifs destinés notamment à la prévention et/ou au traitement des affections cutanées.

En outre, la composition de l'invention peut contenir de façon avantageuse une eau thermale et/ou minérale, notamment choisie parmi l'eau de Vittel, les eaux du bassin de Vichy et l'eau de la Roche Posay.

Selon un autre de ses objets, la présente invention se rapporte à un procédé de traitement non thérapeutique du chevelu fragilisé, sensibilisé, stressé ou réactif qui peut être mis en oeuvre notamment en administrant les compositions cosmétiques telles que définies ci-dessus, par voie orale.

Le procédé cosmétique selon l'invention peut être mis en oeuvre par administration orale journalière d'une composition formulée par exemple sous forme de gélules, gels, lotions, dragées, émulsions, comprimés, capsules ou ampoules buvables, en quantité et nombre adéquats, selon leur forme.

Le procédé selon l'invention peut comprendre une administration unique. Selon un autre mode de réalisation, l'administration est répétée par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

Dans la description et dans les exemples suivants, sauf indication contraire, les pourcentages sont des pourcentages en poids et les plages de valeurs libellées sous la forme « entre ... et ... » incluent les bornes inférieure et supérieure précisées. Les ingrédients sont mélangés, avant leur mise en forme, dans l'ordre et dans des conditions facilement déterminées par l'homme de l'art.

Les exemples ci-après sont présentés à titre illustratif et non limitatif du domaine de l'invention.

### Exemple 1 - compositions pour la voie orale

**Exemple 1 a : Stick poudre**

| **Principe actif** | Par dragée |
|---|---|
| *Lactobacillus paracaseï ST11* | 10¹⁰ ufc |
| *Bifidobacterium lactis Bb12* | 10¹⁰ ufc |
| Citrate de calcium | 50 mg |
| Acide petroselinique | 100 mg |

| **Excipient** | |
|---|---|
| Gomme de xanthane | 0,8 mg |
| Benzoate de sodium | 0,2 mg |
| Maltodextrine | qsp 30g |

On peut prendre un stick par jour.

**Exemple 1b : formulation de type dragée**

| **Matières actives** | mg/dragée |
|---|---|
| Gluconate de magnésium | 50 |
| *Lactobacillus paracaseï ST11* | 5,10⁸ ufc |
| *Bifidobacterium lactis Bb12* | 5,10⁸ ufc |
| Citrate de calcium | 200 |
| Huile de coriandre | 250 |
| | |

| **Excipient du noyau de la dragée** | |
|---|---|
| Cellulose micro-cristalline | 70 |
| Encompress™ | 60 |
| Stéarate de magnésium | 3 |
| Silice colloïdale anhydre | 1 |
| | |

| **Agent d'enrobage** | |
|---|---|
| Gomme laque | 5 |
| Talc | 61 |
| Saccharose | 250 |
| Polyvidone | 6 |
| Dioxyde de titane | 0,3 |
| Agent de coloration | 5 |

Ce type de dragée peut être pris 1 à 3 fois par jour.

## Revendications

1. Utilisation non thérapeutique d'au moins l'acide petrosélinique, de ses sels et/ou esters pour renforcer la cohésion du tissu cutané du cuir chevelu et prévenir et/ou traiter des cuirs chevelus fragilisés, sensibilisés et/ou réactifs, **caractérisée en ce que** ledit acide pétrosélinique est administré par voie orale.

2. Utilisation selon la revendication1, pour lisser la surface du cuir chevelu.

3. Utilisation selon la revendication 1, pour renforcer la résistance mécanique du cuir chevelu.

4. Utilisation selon la revendication 3, pour renforcer la résistance du cuir chevelu vis-à-vis des agressions extérieures choisies parmi les facteurs environnementaux de type agents irritants ou polluants ; sollicitations mécaniques ; déséquilibres thermiques ou climatiques ; xénobiotiques ; traitements chimiques cosmétiques ou dermatologiques ou par le facteur physiologique stress.

5. Utilisation selon l'une quelconque des revendications précédentes, **caractérisée en ce que** l'acide pétrosélinique est utilisé sous forme isolée ou dans un extrait végétal.

6. Utilisation selon l'une quelconque des revendications précédentes, **caractérisée en ce que** la teneur en acide pétrosélinique, son sel et/ou son ester, est telle que la dose journalière est comprise entre 0,5 et 2500 mg/jour.

7. Utilisation selon l'une quelconque des revendications précédentes, **caractérisée en ce que** l'acide pétrosélinique est utilisé sous forme d'huile d'ombellifère ou de Géranium sanguineum.

8. Utilisation selon la revendication 7, **caractérisée en ce que** l'huile d'ombellifère est choisie parmi les huiles de graines de coriandre, cerfeuil, carotte, céleri, cumin, carvi, persil et aneth.

9. Utilisation selon l'une quelconque des revendications précédentes, **caractérisée en ce que** ledit acide pétrosélinique est utilisé en association avec au moins un actif choisi parmi les vitamines B3, B5, B6, B8, C, E, ou PP, les caroténoïdes, les curcuminoïdes, la niacine, les flavonoïdes, un ou plusieurs cation(s) minéral(aux) divalent(s), les extraits bactériens de bactéries filamenteuses non photosynthétiques et non fructifiantes, les probiotiques, prébiotiques ou un mélange de probiotiques et/ou un mélange de prébiotiques.

10. Procédé non thérapeutique et cosmétique de traitement des cuirs chevelus fragilisés, sensibilisés, stressés et/ou réactifs comprenant l'administration orale d'une composition comprenant au moins l'acide pétrosélinique.

## Patentansprüche

1. Nichttherapeutische Verwendung von mindestens Petroselinsäure, ihren Salzen und/oder Estern zur Verstärkung der Haftung des Hautgewebes der Kopfhaut und zur Vorbeugung und/oder Behandlung von geschwächten, empfindlichen und/oder reaktiven Kopfhäuten, **dadurch gekennzeichnet, dass** die Petroselinsäure oral verabreicht wird.

2. Verwendung nach Anspruch 1, zum Glätten der Oberfläche der Kopfhaut.

3. Verwendung nach Anspruch 1, zum Verstärken der mechanischen Widerstandsfähigkeit der Kopfhaut.

4. Verwendung nach Anspruch 3, zur Verstärkung der mechanischen Widerstandsfähigkeit der Kopfhaut gegenüber äußeren Angriffen, ausgewählt aus Umweltfaktoren in der Art von reizenden oder verschmutzenden Mitteln; mechanischen Beanspruchungen; thermischen oder klimatischen Ungleichgewichten; Xenobiotika; chemischen, kosmetischen oder dermatologischen Behandlungen oder durch physiologischen Stressfaktor.

5. Verwendung nach einem der vorangehenden Ansprüche, **dadurch gekennzeichnet, dass** die Petroselinsäure in isolierter Form oder in einem Pflanzenextrakt verwendet wird.

6. Verwendung nach einem der vorangehenden Ansprüche, **dadurch gekennzeichnet, dass** der Gehalt an Petroselinsäure, ihres Salzes und/oder ihres Esters so ist, dass die tägliche Dosis von 0,5 bis 2500 mg/Tag beträgt.

7. Verwendung nach einem der vorangehenden Ansprüche, **dadurch gekennzeichnet, dass** die Petroselinsäure in Form von Doldenblütleröl oder Geranium-sanguineum-Öl verwendet wird.

8. Verwendung nach Anspruch 7, **dadurch gekennzeichnet, dass** das Doldenblütleröl ausgewählt ist aus den Ölen von Koriandersamen, Kerbel, Karotte, Sellerie, Schwarzkümmel, Kümmel, Petersilie und Dill.

9. Verwendung nach einem der vorangehenden Ansprüche, **dadurch gekennzeichnet, dass** die Petroselinsäure in Verbindung mit mindestens einem Wirkstoff verwendet wird, der ausgewählt ist aus Vitaminen B3, B5, B6, B8, C, E oder PP, Carotinoiden, Curcuminoiden, Niacin, Flavonoiden, einem oder mehreren zweiwertigen mineralischen Kation(en), Bakterienextrakten von nicht photosynthetischen und nicht fruchtbildenden Fadenbakterien, Probiotika, Präbiotika oder einem Gemisch von Probiotika und/oder einem Gemisch von Präbiotika.

10. Nichttherapeutisches und kosmetisches Verfahren zur Behandlung von geschwächten, empfindlichen, gestressten und/oder reaktiven Kopfhäuten, umfassend die orale Verabreichung einer Zusammensetzung umfassend mindestens Petroselinsäure.

## Claims

1. The non-therapeutic use of at least petroselinic acid, of its salts and esters for reinforcing cohesion of skin tissue of the scalp and for preventing and/or treating weakened, sensitized, and/or reactive scalps, **characterized in that** said petroselinic acid is administered orally.

2. The use according to claim 1, for smoothing the surface of the scalp.

3. The use according to claim 1, for reinforcing the mechanical strength of the scalp.

4. The use according to claim 3, for reinforcing the strength of the scalp towards exterior aggressions selected from environmental factors of the irritating or polluting agent type; mechanical stresses; thermal or weather imbalances; xenobiotics; cosmetic or dermatological chemical treatments or by the physiological stress factor.

5. The use according to any of the preceding claims, **characterized in that** petroselinic acid is used in an isolated form or in a plant extract.

6. The use according to any of the preceding claims, **characterized in that** the content of petroselinic acid, its salt and/or its ester is such that the daily dose is comprised between 0.5 and 2,500 mg/day.

7. The use according to any of the preceding claims, **characterized in that** petroselinic acid is used as an oil from umbellifera or *Geranium sanguineum.*

8. The use according to claim 7, **characterized in that** the umbellifera oil is selected from coriander seed oil, chervil, carrot, celery, cumin, carvi, parsley and dill seed oil.

9. The use according to any of the preceding claims, **characterized in that** said petroselinic acid is used in a combination with at least one active selected from among vitamins B3, B5, B6, B8, C, E, or PP, carotenoids, curcuminoids, niacin, flavonoids, one or several mineral divalent cations, bacterial extracts from non-photosynthetic and non-fruiting filamentary bacteria, probiotics, prebiotics or a mixture of probiotics and/or a mixture of prebiotics/

10. A non-therapeutic and cosmetic method for treating weakened, sensitized, stressed and/or reactive scalps comprising the oral administration of a composition comprising at least petroselinic acid.
